# FASCICULE DE BREVET EUROPEEN

(11) **EP 2 366 214 B1**
(45) Date de publication et mention de la délivrance du brevet: **13.02.2013**
(21) Numéro de dépôt: 09803881.3
(22) Date de dépôt: 16.12.2009
(51) Int. Cl.: H01S 3/067, H01S 3/094, A61F 9/01

(54) **LASER IMPULSIONNEL A FIBRE OPTIQUE POUR IMPULSIONS SUB-PICOSECONDE DE HAUTE ENERGIE DANS LA BANDE L ET OUTIL LASER POUR CHIRURGIE OPHTALMIQUE**
GEPULSTER LASER MIT OPTISCHER FASER FÜR HOCHENERGIE-SUB-PICOSEKUNDENIMPULSE IM L-BAND UND LASERWERKZEUG FÜR DIE AUGENCHIRURGIE
PULSED LASER WITH AN OPTICAL FIBRE FOR HIGH-ENERGY SUB-PICOSECOND PULSES IN THE L BAND, AND LASER TOOL FOR EYE SURGERY

(30) Priorité: 17.12.2008 FR 0858687
(43) Date de publication de la demande: 21.09.2011
(73) Titulaire: Centre National de la Recherche Scientifique (C.N.R.S.), 75794 Paris Cedex 16 (FR)
(72) Inventeur: MORIN, Franck, F-91440 Bures Sur Yvette (FR); HANNA, Marc, F-91470 Limours (FR); DRUON, Frédéric, F-91400 Orsay (FR); GEORGES, Patrick, F-78590 Noisy Le Roi (FR)
(74) Mandataire: Chauvin, Vincent
(86) Numéro de dépôt international: PCT/FR2009/052579
(87) Numéro de publication internationale: WO 2010/076511

(56) Documents cités:
- EP-A- 1 087 475
- WO-A1-99/50937
- CA-A1- 2 289 807
- FR-A- 2 745 395
- US-A1- 2001 050 803
- US-A1- 2003 156 605
- US-A1- 2004 176 752
- US-A1- 2005 238 070
- IMESHEV G ET AL: "An optimized Er gain band all-fiber chirped pulse amplification system" OPTICS EXPRESS, OPTICAL SOCIETY OF AMERICA, WASHINGTON, DC, US, vol. 12, no. 26, 27 décembre 2004 (2004-12-27), pages 6508-6514, XP002514375 ISSN: 1094-4087
- BRAUN A M ET AL: "Compact semiconductor-based chirped-pulse amplification laser system" LASERS AND ELECTRO-OPTICS, 2004. (CLEO). CONFERENCE ON SAN FRANCISCO, CA, USA MAY 20-21, 2004, PISCATAWAY, NJ, USA,IEEE, vol. 1, 17 mai 2004 (2004-05-17), pages 912-913, XP010745421 ISBN: 978-1-55752-777-6
- GALVANUSKAS A ET AL: "Robust high-power and wavelength-tunable femtosecond fiber system based on engineerable PPLN devices" NONLINEAR OPTICS '98: MATERIALS, FUNDAMENTALS AND APPLICATIONS TOPICAL MEETING KAUAI, HI, USA 10-14 AUG. 1998, NEW YORK, NY, USA,IEEE, US, 10 août 1998 (1998-08-10), pages 265-267, XP010296376 ISBN: 978-0-7803-4950-6
- MASSICOTT J F ET AL: "HIGH GAIN, BROADBAND, 1.6 M ER3+ DOPED SILICA FIBRE AMPLIFIER" ELECTRONICS LETTERS, IEE STEVENAGE, GB, vol. 26, no. 20, 27 septembre 1990 (1990-09-27), page 1645/1646, XP000109503 ISSN: 0013-5194

## Description

La présente invention concerne un laser impulsionnel à fibre optique apte à générer des impulsions ultra-brèves et de forte énergie à une cadence élevée et dont la longueur d'onde d'émission se situe dans une fenêtre de transparence de la cornée.

Plus précisément l'invention concerne un laser à fibre optique et à dérive de fréquence pour l'amplification d'impulsions sub-picoseconde de forte énergie et dont la longueur d'onde d'émission est comprise entre 1565 nm et 1625 nm.

Un tel laser permet de générer des impulsions laser de forte énergie, c'est à dire dans la suite de ce document dont l'énergie est comprise entre 100 nanojoules (nJ) par pulse et 100 microjoules (µJ) par impulsions, avec un taux de répétition élevé (10kHz - 1 MHz) et présentant une bonne qualité spatiale de faisceau de sortie (proche d'un faisceau gaussien, M² < 1.5).

Le laser de l'invention est essentiellement constitué de fibres optiques, ce qui le rend très robuste et intégrable dans des dispositifs compacts.

L'invention concerne également un outil de chirurgie ophtalmique utilisant un tel laser notamment pour la découpe profonde de cornée ou pour le traitement du glaucome.

Les lasers commencent à être utilisés dans la découpe de cornée à la place d'outils mécaniques tels le microkératome. La profondeur de découpe va jusqu'à un millimètre et doit être très précisément contrôlée. Dans les opérations de découpe laser, une série d'impulsions laser est focalisée suivant la ligne de découpe souhaitée. L'oeil du patient est immobilisé pendant toute la durée de l'intervention, qui doit donc être aussi courte que possible. Les lasers utilisés pour la découpe de cornée doivent donc être aptes à générer des impulsions avec une cadence très élevée. Dans l'application de découpe de cornée, il est essentiel de disposer d'impulsions à la fois de courte durée, de haute énergie, de bonne qualité spatiale et qui soient peu absorbées et/ou diffusées par les tissus sains ou malades dans lesquels le faisceau laser est focalisé. La qualité de la découpe dépend de la qualité spatiale et temporelle des impulsions laser, de leur énergie, et de leur focalisation. Un laser permet une découpe plus précise et plus complexe qu'un microkératome.

Cependant, les lasers existants ne permettent pas une telle découpe rapide du fait d'une énergie par impulsion insuffisante et/ou à cause de la diffusion et des déformations du faisceau laser à travers les milieux optiques d'un oeil pathologique qui détériorent la focalisation du faisceau.

Des lasers impulsionnels à fibre optique ont été utilisés dans la fabrication d'outils pour le traitement médical ou cosmétique. Le document US 6,723,090 (Altshuler et al.) décrit un laser à fibre comprenant une diode de pompe et une section de fibre optique amplificatrice. Ce laser de faible énergie est utilisé dans des traitements dermatologiques ou médicaux à une longueur d'onde accordable pour être absorbée par les tissus. Un tel laser peut fonctionner en mode impulsionnel déclenché. Toutefois, la durée minimum des impulsions est d'au moins 10 ms et le taux de répétition des impulsions est limité. Un tel laser n'est pas apte à produire des impulsions sub-picosecondes de forte énergie avec un taux de répétition élevé.

Par ailleurs, des lasers à fibre optique capables de générer des impulsions femtoseconde d'énergie élevée avec des taux de répétition importants ont été développés récemment. Des lasers à fibre basés sur la technologie d'amplificateurs à dérive de fréquence (CPA) permettent de limiter les effets non linéaires apparaissant lors de l'amplification des impulsions dans les fibres et ainsi d'obtenir des impulsions de forte énergie et de faible durée.

Le document US 7,131,968 (Bendett et al.) décrit un laser à fibre pour la chirurgie ophtalmique et plus particulièrement pour la découpe rapide de cornée dans des opérations de correction de réfraction. Ce laser est un laser à fibre à dérive de fréquence comprenant un oscillateur, un étireur à fibre, un préamplificateur, un amplificateur de puissance et un compresseur. Ce laser génère des impulsions femtoseconde avec un taux de répétition élevé (50 à 100 kHz). Comme la plupart des lasers à fibre dopée Ytterbium, ce dispositif émet des impulsions à la longueur d'onde de 1,05 µm.

Or la cornée oedémateuse est fortement diffusante pour les longueurs d'onde proches de 1 µm. L'énergie des impulsions laser n'est donc plus suffisante au point de focalisation pour assurer une découpe efficace. De tels lasers ne sont pas adaptés à la découpe rapide, précise et fiable de cornées pathologiques.

Il n'existe pas à ce jour de laser sub-picoseconde, à base de fibres dopées Erbium, suffisamment énergétique pour la découpe de cornée et fonctionnant dans le domaine de longueur d'onde compris entre 1565 nm et 1625 nm.

Les amplificateurs à fibres optiques dopés Erbium pompés par des diodes lasers à 980 nm ou 1480 nm présentent certes une bande d'émission de 1565 à 1625 nm (dite bande L en télécommunications). Toutefois, pour la bande L, la longueur de fibre optique amplificatrice doit être beaucoup plus importante que pour la bande C (entre 1530 nm et 1565 nm). Les impulsions se propageant sur une grande longueur de fibre subissent des effets non linéaires qui déforment ces impulsions temporellement. De plus, le gain spectral d'un amplificateur en bande L pompé par une diode laser à 980 nm ou 1480 nm varie fortement le long de la fibre amplificatrice. Un tel amplificateur ne permet pas de générer des impulsions sub-picoseconde et de forte énergie dans la bande L.

La présente invention a pour but de remédier à ces inconvénients et concerne plus particulièrement un laser impulsionnel à fibre optique et à dérive de fréquence pour l'amplification d'impulsions sub-picoseconde de bonne qualité spatiale et de forte énergie comprenant un oscillateur, un étireur à fibre, au moins un réducteur de cadence, un ou plusieurs étages de pré-amplification, un étage d'amplification de puissance et un compresseur. L'oscillateur est apte à émettre des impulsions lumineuses de durée sub-picoseconde, dont l'énergie est comprise entre 10 pJ et 10 nJ et dont la longueur d'onde d'émission est comprise entre 1565 à 1625 nm. L'étireur à fibre est apte à étirer temporellement ces impulsions lumineuses. Chaque étage de préamplification comprend une section de fibre optique dopée Erbium ou co-dopée Erbium-Ytterbium et une pompe apte à pomper optiquement la section de fibre optique de préamplification. Le (ou les) réducteur de cadence est apte à réduire le taux de répétition des impulsions laser de sortie entre 10 kHz et 1 MHz. L'étage d'amplification de puissance comprend également une section de fibre optique dopée Erbium ou co-dopée Erbium-Ytterbium et une pompe apte à pomper optiquement cette section de fibre optique. En sortie de l'étage amplificateur de puissance, un compresseur est apte à recomprimer temporellement les impulsions lumineuses amplifiées. Selon l'invention, la pompe de l'amplificateur de puissance génère au moins une longueur d'onde de pompe (λ_{P}) dans la bande de longueurs d'onde comprise entre 1530 et 1565 nm de manière à obtenir des impulsions de sortie du laser dont la longueur d'onde d'émission est comprise entre 1565 nm et 1625 nm et dont l'énergie est comprise entre 100 nJ et 100 µJ.

Selon un mode de réalisation de l'invention, la section de fibre optique de l'étage d'amplification de puissance est une fibre à large coeur (LMA large mode area).

Selon un mode de réalisation particulier de l'invention, le faisceau en sortie est de bonne qualité spatiale, de coefficient M² inférieur à 1,5.

Selon un mode de réalisation particulier de l'invention, la pompe de l'amplificateur de puissance est un laser à fibre optique dopée erbium ou co-dopée erbium-ytterbium.

Selon un mode de réalisation particulier de l'invention, la pompe de l'amplificateur de puissance est couplée directement dans le coeur dopé de la fibre optique amplificatrice de puissance.

Selon un mode de réalisation particulier de l'invention, la pompe de l'amplificateur de puissance est apte à pomper la section de fibre optique de l'amplificateur de puissance de manière co-propagative et/ou contra-propagative.

Selon un mode de réalisation particulier de l'invention, la pompe de l'amplificateur de puissance est apte à générer au moins une seconde longueur d'onde de pompe (λ_{P}) comprise entre 1530 nm et 1565 nm.

Selon un mode de réalisation particulier de l'invention, la longueur d'onde (λ_{P}) de la pompe de l'amplificateur de puissance est ajustable de manière à optimiser spectralement et temporellement les impulsions de sortie.
Selon un mode de réalisation particulier de l'invention, la pompe de l'amplificateur de puissance est un laser à fibre optique.

Selon un mode de réalisation particulier de l'invention, le laser comprend une autre pompe apte à pomper optiquement la section de fibre optique amplificatrice de puissance à une autre longueur d'onde de pompe λ'_{P} comprise entre 970 et 990 nm simultanément avec la première pompe à la longueur d'onde de pompe λ_{P} de manière à augmenter le gain d'amplification dans la section de fibre optique amplificatrice.

L'invention concerne également un outil laser pour chirurgie ophtalmique comprenant un laser impulsionnel à fibre optique selon l'un des modes de réalisation décrits. Selon un mode de réalisation particulier de l'outil laser pour chirurgie ophtalmique de l'invention, l'énergie des impulsions laser est supérieure à 100 nJ.

Les caractéristiques d'un tel laser ci-dessus énoncées permettent de disposer d'un laser générant des impulsions laser sub-picoseconde d'énergie comprise entre 100 nJ et 100 µJ et dont la longueur d'onde est située entre 1565 et 1625 nm.

Une première application particulièrement avantageuse du laser impulsionnel sub-picoseconde, haute énergie et bande L de l'invention concerne un outil laser de chirurgie ophtalmique pour la découpe profonde de cornée dans l'oeil humain ou animal.

Dans ce texte, on entend par sub-picoseconde des impulsions dont la durée est généralement inférieure à la picoseconde, et peut s'étendre jusqu'à 1 ou 2 picoseconde. Et on entend par impulsions femtoseconde, des impulsions dont la durée s'étend de 1 à plusieurs centaines de femtosecondes.

La présente invention concerne également les caractéristiques qui ressortiront au cours de la description qui va suivre et qui devront être considérées isolément ou selon toutes leurs combinaisons techniquement possibles.

Cette description est donnée à titre d'exemple non limitatif fera mieux comprendre comment l'invention peut être réalisée en référence aux dessins annexés sur lesquels :
- la figure 1 représente un mode de réalisation d'un dispositif de l'invention ;
- la figure 2 représente des courbes de puissance moyenne en fonction de la longueur de fibre amplificatrice pour deux longueurs d'onde de pompe (λ_{P}) ;
- la figure 3 représente des courbes de gain spectral (α) pour différentes longueurs d'onde de pompe (α_{P}) et longueurs de fibre (L_{F}) de l'amplificateur de puissance.

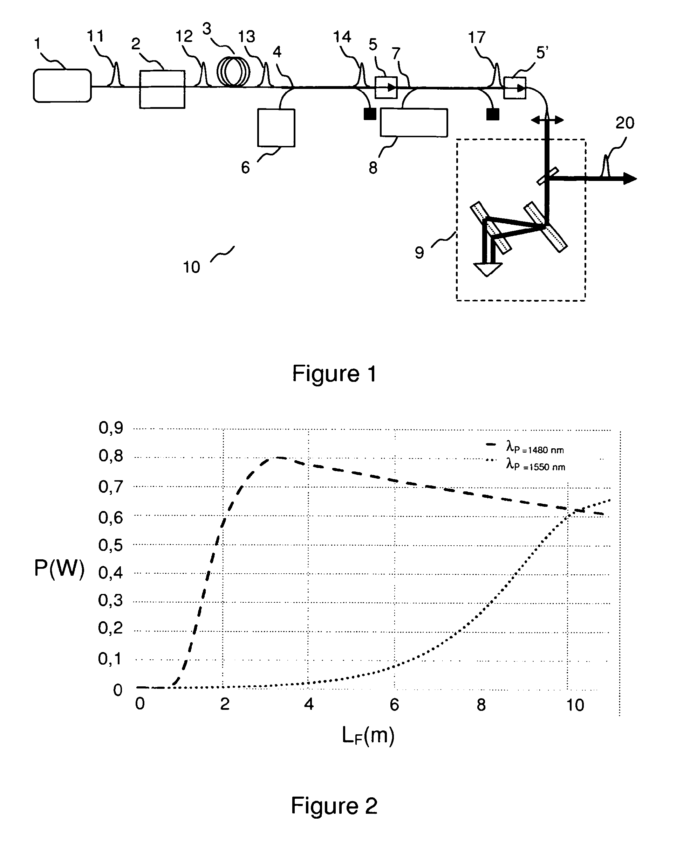

Selon le mode de réalisation préféré représenté schématiquement Figure 1, le laser impulsionnel 10 de l'invention est constitué de plusieurs parties : un oscillateur 1 produisant des impulsions sub-picoseconde, un réducteur de cadence 2 de type modulateur acousto-optique ou électro-optique, un étireur d'impulsions 3, un étage de préamplification à fibre optique 4, un étage d'amplification de puissance à fibre optique 7, et un compresseur d'impulsions 9.

Le laser 10 est essentiellement constitué de composants fibrées, dont la partie active est une fibre dopée Er ou co-dopée Er-Yb. L'oscillateur 1 émet des impulsions lumineuses 11 de durée sub-picoseconde et dont la longueur d'onde centrale est comprise entre 1565 et 1625 nm. Les fonctions de compensation de dispersion et de verrouillage de mode sont réalisées en optique guidée. Le verrouillage de mode peut être actif, avec l'utilisation de modulateurs électro-optiques, ou passif, exploitant par exemple l'effet de rotation de polarisation non linéaire ou d'un miroir de Bragg absorbant saturable.

Le réducteur de cadence 2 est constitué d'un modulateur électro-optique ou acousto-optique, qui peut-être massif (faisceaux en espace libre) ou intégré et connectorisé à des fibres optiques. Le réducteur de cadence 2 permet d'ajuster la cadence de répétition des impulsions selon la tâche à effectuer. La cadence et/ou le nombre d'impulsions par salve seront optimisés selon le type d'application.

L'étireur 3 est un système dispersif qui peut être réalisé par un tronçon de fibre optique à dispersion fortement normale (DCF pour Dispersion Compensating Fiber), d'une longueur ajustée afin d'obtenir l'étirement désiré. L'étireur peut également être réalisé à l'aide d'une combinaison de composants optiques massifs comme des prismes et réseaux de diffraction ainsi que des lentilles et miroirs pour réaliser le système d'imagerie optique. Ce système sert à allonger la durée des impulsions initiales 11 afin de limiter les effets non-linéaires dans les étages de préamplification et d'amplification de puissance.

Les étages de préamplification et d'amplification de puissance sont à base de fibres 4 respectivement 7 dopées Erbium ou co-dopées Er-Yb. Les étages de préamplification peuvent être pompés par des diodes mono émetteur 6 couplées dans le coeur monomode de la fibre amplificatrice 4. Le dispositif comprend également des isolateurs optiques 5, 5'. L'étage de puissance présente la principale originalité du système, et est décrit ci-après.

Le compresseur 9 est un dispositif dispersif réalisant (au premier ordre) la fonction de dispersion inverse de celle de l'étireur. Plus précisément, le compresseur prend aussi en compte la compensation de la dispersion se produisant dans les différents amplificateurs. Ce compresseur 9 peut être, tout comme l'étireur 3, réalisé à l'aide de composants fibrés ou d'une combinaison de composants optiques massifs comme des prismes et réseaux de diffraction. Pour affiner la compression, le compresseur 9 peut aussi contenir des composants massifs, voire des miroirs diélectriques à pas variable (« chirped »). Le compresseur 9 recomprime temporellement les impulsions lumineuses amplifiées 17 afin de générer des impulsions laser de sortie 20 amplifiées et comprimées temporellement.

L'étage de puissance constitue le coeur du système car il doit répondre à des critères antagonistes :
- d'une part, son architecture doit limiter le plus possible les effets non linéaires afin d'éviter toute dégradation temporelle des impulsions sub-picoseconde. Ceci tend à favoriser les courtes longueurs de fibre et les faibles puissances crêtes.
- d'autre part il doit présenter un gain plat sur plusieurs dizaines de nanomètres, centré autour de 1590 nm. Cette caractéristique nécessite, pour les amplificateurs à fibre dopée Er ou co-dopée Er-Yb, une faible inversion de population moyenne dans la fibre, et donc une grande longueur de fibre pour avoir un gain suffisant.

La section de fibre optique amplificatrice 7 de l'étage de puissance peut être pompée directement dans le coeur dopé par un laser monomode, et non à travers la gaine. Ce pompage dans le coeur permet de réduire la puissance nécessaire pour saturer l'absorption de la pompe. D'autre part, au lieu d'utiliser classiquement des diodes lasers dont les longueurs d'onde de pompe sont de 980 nm ou 1480 nm, la longueur d'onde λ_{P} de la pompe 8 de la section de fibre d'amplification de puissance est choisie pour être comprise entre 1530 et 1565 nm. Cette longueur d'onde de pompe λ_{P} permet avantageusement de fixer l'inversion de population à une valeur constante le long de la fibre. Cette longueur d'onde de pompe λ_{P} proche de la longueur d'onde d'émission permet également de réduire le bruit d'émission spontanée amplifiée de longueur d'onde inférieure à la longueur d'onde de pompe λ_{P} ainsi que d'augmenter sensiblement le rendement énergétique entre pompe et signal.

Une pompe fonctionnant à une longueur d'onde λ_{P} proche de 1550 nm peut être réalisée au moyen d'un laser à fibre co-dopée Er-Yb.

Il est apparu que le pompage de l'étage amplificateur de puissance dans la bande 1530 - 1565 nm présente l'avantage majeur de fixer l'inversion de population à une valeur constante le long de la fibre. En effet, pour une longueur de fibre suffisante un pompage à 1480 ou 980 nm permet également d'obtenir une courbe de gain spectral similaire en sortie de fibre. Toutefois dans ces cas, la dépendance spectrale du gain varie beaucoup le long de la fibre, avec un maximum se déplaçant des basses vers les hautes longueurs d'onde. Ceci entraine un gain fort en début de fibre, puis un filtrage progressif des courtes longueurs d'onde. Le résultat est que la puissance crête intégrée sur la longueur de la fibre, qui détermine l'intégrale B responsable des effets non linéaires, est trois fois plus grande pour un pompage à 1480 ou 980 nm que dans le cas du pompage à 1550 nm. La figure 2 représente deux courbes de puissance du signal de sortie en fonction de la longueur L_{F} de la section de fibre optique 7 amplificatrice de puissance respectivement pour une longueur d'onde de pompe λ_{P} classique de 1480 nm. et pour une longueur d'onde de pompe λ_{P} conformément à l'invention de 1550 nm. Cette figure 2 montre tout l'intérêt de pomper l'étage amplificateur de puissance à une longueur d'onde λ_{P} ~ 1550 nm pour minimiser les effets non linéaires. Pour une longueur L_{F} d'environ 10 m. on obtient une puissance de sortie équivalente sur les deux courbes, mais l'intégrale B de la courbe correspondant à une longueur d'onde de pompe λ_{P} de 1550 nm apparaît visiblement très inférieure à celle de la courbe correspondant à une longueur d'onde de pompe λ_{P} de 1480 nm.

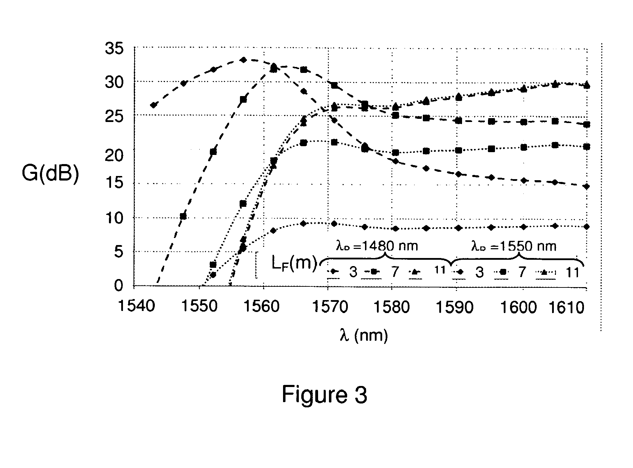

L'autre avantage notable du pompage à environ 1550 nm est la relative insensibilité de la forme de la courbe de gain spectral aux variations de puissance de pompe et de longueur de fibre, ce qui contribue à la robustesse du système et permet d'ajuster facilement l'énergie des impulsions sans changer leurs caractéristiques spectrales, comme illustré en figure 3. La figure 3 représente des courbes de gain spectral (α) obtenues respectivement pour différentes longueurs L_{F} de section de fibre optique 7 amplificatrice de puissance et pour différentes longueur d'onde de pompe λ_{P}. Les courbes de gain spectral (α) à une longueur d'onde de pompe λ_{P} de 1550 nm sont plates ou quasi-plates sur une large plage spectrale et leur niveau augmente en fonction de la longueur L_{F} de la fibre amplificatrice 7, contrairement aux courbes de gain spectral pour une longueur d'onde de pompe λ_{P} de 1480 nm. Selon un mode de réalisation préféré, la section de fibre optique amplificatrice 7 est une fibre optique à grande aire effective.

Selon un mode de réalisation particulièrement avantageux, la fibre amplificatrice est pompée simultanément par deux pompes : une pompe à 1550 nm et une pompe à 980 nm. Cette combinaison de deux longueurs d'onde de pompe permet d'obtenir à la fois un fort gain d'amplification et de faibles non linéarités. On observe en effet que le pompage à 1550 nm permet de supprimer l'émission spontanée amplifiée (ASE) générée par un pompage à 980 nm et ainsi d'augmenter l'énergie des impulsions laser en sortie à ~1600 nm. Ce double pompage permet d'augmenter le gain de 33% comparé à un pompage unique à 980 nm. On peut ainsi obtenir des impulsions laser de 650 fs dont l'énergie est de 2.2 µJ par impulsion à une cadence de 100 kHz.

D'autres caractéristiques de l'invention permettent de réduire les effets non linéaires pour atteindre les performances reportées dans le tableau 1.

**Tab. 1 : Gammes de fonctionnement du laser en sortie de divers composants**

| | Oscillateur | Pulse picker | Etireur | Pré-amplificateurs | Amplificateur de puissance | Compresseur |
|---|---|---|---|---|---|---|
| Energie | ~2 nJ | ~1 nJ | ~500 pJ | 10-20 nJ | 200nJ-200 µJ | 100nJ - 100 µJ |
| Cadence | ~50 MHz | 10-1000 kHz | 10-1000 kHz | 10-1000 kHz | 10-1000 kHz | 10-1000 kHz |
| Puissance moyenne | ~100 mW | 10-1000 µW | 5-500 µW | 0.1-20 mW | 2mW-200W | 1mw-100 W |
| Puissance crête | ~20 kW | ~10 kW | ~1 W | 20-40 W | 400W-400 kW | 50 kW-1 GW |
| Durée des impulsions | ~100 fs | ~100 fs | ~500 ps | ~500 ps | 500 ps | 100-2000 fs |

On obtient un laser 10 permettant de générer des impulsions laser 20 sub-picoseconde d'énergie comprise entre 100 nJ et 100 µJ par impulsions et dont la longueur d'onde est située entre 1565 et 1625 nm. Les caractéristiques du laser de l'invention permettent d'atteindre les performances suivantes : cadence comprise entre 10 kHz et 1 MHz, énergie par impulsion de 100 nJ à 100 µJ, durée d'impulsion comprise entre 100 fs et 2 ps. Les performances nominales sont des impulsions de 500 fs, dont l'énergie par pulse est de 5 µJ, à une fréquence de répétition de 300 kHz.

Le laser à impulsions courtes de l'invention est destiné à être intégré dans un système de chirurgie de l'oeil adapté à la découpe des cornées en profondeur, notamment pathologiques, pour la réalisation de greffes totales ou partielles. Ce laser 10 est essentiellement basé sur la technologie des fibres optiques, à l'exception du compresseur final. Les amplificateurs à fibre sont basés sur la technologie erbium. Ce laser 10 émet dans la gamme de longueur d'onde de 1570 nm à 1610 nm, l'optimum étant 1590 nm afin de bénéficier d'une fenêtre de transparence de la cornée tout en minimisant l'impact de la forte diffusion optique des tissus pathologiques.

On obtient un laser sub-picoseconde à la longueur d'onde de 1590 nm (±20 nm) adaptée à la découpe rapide de cornées en profondeur. Ce laser est particulièrement adapté aux cornées pathologiques qui sont généralement diffusantes. La longueur d'onde élevée du rayonnement permet d'atteindre une zone profonde (1 mm) de la cornée à découper en subissant peu de diffusion. Les impulsions générées ont une durée inférieure à 1 ps et une énergie comprise entre 100 nJ et 100 µJ, ce qui correspond à un régime de découpe athermique. Le laser a un taux de répétition suffisant (~100 kHz) pour réaliser la découpe rapidement.

Les éléments constituant le laser de l'invention sont essentiellement à base de fibre optique, ce qui rend le dispositif robuste, et permet de l'intégrer facilement dans un environnement médical. Dans l'exemple de réalisation de l'invention, la longueur L_{F} de fibre optique nécessaire à l'amplification de puissance est de l'ordre de quelques mètres à une dizaine de mètres. Malgré cette longueur le laser de l'invention permet de limiter les effets non linéaires dans la fibre amplificatrice de puissance.

Une première application du laser impulsionnel sub-picoseconde, haute énergie et bande L de l'invention concerne un outil laser de chirurgie ophtalmique pour la découpe profonde de cornée dans l'oeil humain ou animal.

Le laser de l'invention permet une découpe rapide et de bonne qualité de la cornée d'un patient pour un traitement de cette cornée ou pour une greffe de cornée. L'amélioration de la qualité de découpe permet d'une part de retirer plus facilement la cornée à traiter ou à remplacer et d'autre part permet une réimplantation de la nouvelle cornée et une cicatrisation de meilleure qualité avec moins de complication après le traitement ou la greffe.

Une autre application de l'invention en chirurgie ophtalmique est un laser pour le traitement du glaucome.

## Revendications

1. Laser impulsionnel (10) à fibre optique et à dérive de fréquence pour l'amplification d'impulsions sub-picoseconde, dont la durée est inférieure à 2 picosecondes, de bonne qualité spatiale et de forte énergie comprenant :
- un oscillateur (1) apte à émettre des impulsions lumineuses (11) de durée sub-picoseconde et dont la longueur d'onde d'émission est comprise entre 1565 et 1625 nm ;
- un étireur à fibre (3) apte à étirer temporellement les impulsions lumineuses (11) ;
- un ou plusieurs étages de pré-amplification comprenant chacun une section de fibre optique (4) dopée Erbium ou co-dopée Erbium-Ytterbium et une pompe (6) apte à pomper optiquement la section de fibre optique (4) de préamplification ;
- au moins un réducteur de cadence (2) apte à réduire le taux de répétition des impulsions laser de sortie (20) entre 10kHz et 1 MHz ;
- un étage d'amplification de puissance comprenant une section de fibre optique (7) dopée Erbium ou co-dopée Erbium-Ytterbium et une première pompe (8) apte à pomper optiquement la section de fibre optique (7) amplificatrice de puissance, ledit étage d'amplification de puissance étant apte à produire des impulsions amplifiées (17) ;
- un compresseur (9) apte recomprimer temporellement les impulsions lumineuses amplifiées (17) en des impulsions laser de sortie (20) ;
**caractérisée en ce que** :
- ladite première pompe (8) de la fibre optique (7) amplificatrice de puissance est apte à générer au moins une longueur d'onde de pompe λ_{P} comprise entre 1530 et 1565 nm de manière à obtenir des impulsions de sortie (20) dont la longueur d'onde d'émission est comprise entre 1565 et 1625 nm et dont l'énergie est comprise entre 100 nJ et 100 µJ.

2. Laser impulsionnel (10) selon la revendication 1, dans lequel la longueur L_{F} de la section de fibre optique (7) de l'étage d'amplification de puissance est inférieure à une dizaine de mètres.

3. Laser impulsionnel (10) selon la revendication 1 ou la revendication 2, **caractérisé en ce que** la section de fibre optique (7) de l'étage d'amplification de puissance est une fibre à large coeur (LMA large mode area).

4. Laser impulsionnel selon l'une des revendications 1 à 3, **caractérisé en ce que** le coeur de la fibre optique (4) de préamplification est monomode, de manière à ce que le faisceau en sortie soit de bonne qualité spatiale, de coefficient M² inférieur à 1,5.

5. Laser impulsionnel selon l'une des revendications 1 à 4, **caractérisé en ce que** la première pompe (8) de l'amplificateur de puissance est un laser à fibre optique dopée erbium ou co-dopée erbium-ytterbium.

6. Laser impulsionnel selon l'une des revendications 1 à 5, **caractérisé en ce que** la première pompe (8) de l'amplificateur de puissance est couplée directement dans le coeur dopé de la fibre optique (7) amplificatrice de puissance.

7. Laser impulsionnel selon l'une des revendications 1 à 6, **caractérisé en ce que** la première pompe (8) de l'amplificateur de puissance est apte à pomper la section de fibre optique (7) de l'amplificateur de puissance de manière co propagative et/ou contra-propagative.

8. Laser impulsionnel selon l'une des revendications 1 à 7, **caractérisé en ce que** la première pompe (8) de l'amplificateur de puissance est apte à générer au moins une seconde longueur d'onde de pompe comprise entre 1530 nm et 1565 nm.

9. Laser impulsionnel selon l'une des revendications 1 à 8, **caractérisé en ce que** la longueur d'onde λ_{P} de la première pompe (8) de l'amplificateur de puissance est ajustable de manière à optimiser spectralement et temporellement les impulsions de sortie.

10. Laser impulsionnel selon l'une des revendications 1 à 9, **caractérisé en ce qu'**il comprend en outre une autre pompe apte à pomper optiquement la section de fibre optique (7) amplificatrice de puissance à une autre longueur d'onde de pompe λ'_{P} comprise entre 970 et 990 nm simultanément avec la première pompe (8) à la longueur d'onde de pompe λ_{P} de manière à augmenter le gain d'amplification dans la section de fibre optique amplificatrice.

11. Outil laser pour chirurgie ophtalmique comprenant un laser impulsionnel à fibre optique selon l'une des revendications 1 à 10.

12. Outil laser selon la revendication 11, **caractérisé en ce que** l'énergie des impulsions laser (20) est supérieure à 100 nJ.

## Claims

1. A chirped pulse amplification fiber laser (10) for amplifying sub-picosecond pulses, which duration is less than two picoseconds, of good spatial quality and high energy, the laser comprising:
• an oscillator (1) suitable for emitting light pulses (11) of sub-picosecond duration and of emission wavelengths lying in the range 1565 nm to 1625 nm;
• a fiber pulse stretcher (3) suitable for stretching the light pulses (11) in time;
• one or more pre-amplification stages, each comprising a section of erbium-doped or erbium-ytterbium co-doped optical fiber (4) and a pump (6) suitable for optically pumping the section of pre-amplification optical fiber (4);
• at least one rate reducer (2) suitable for reducing the repetition rate of the output laser pulses (20) to lie in the range 10 kHz to 1 MHz;
• a power amplifier stage comprising a section of erbium-doped or erbium-ytterbium co-doped optical fiber (7) and a first pump (8) suitable for optically pumping the section of power amplifier optical fiber (7), said power amplifier stage being suitable for producing amplified pulses (17);
• a pulse compressor (9) suitable for recompressing the amplified light pulses (17) in time to produce output laser pulses (20);
the laser being **characterized in that**:
• said first pump (8) of the power amplifier optical fiber (7) is suitable for generating at least one pump wavelength λ_{P} lying in the range 1530 nm to 1565 nm so as to obtain output pulses (20) of emission wavelength lying in the range 1565 nm to 1625 nm, and of energy lying in the range 100 nJ to 100 µJ.

2. A pulsed laser (10) according to claim 1, wherein the length L_{F} of the section of power amplifier optical fiber (7) is less than about 10 meters.

3. A pulsed laser (10) according to claim 1 or claim 2, **characterized in that** the section of optical fiber (7) of the power amplifier stage is a large mode area (LMA) fiber.

4. A pulsed laser according to any one of claims 1 to 3, **characterized in that** the core of the pre-amplification optical fiber (4) is monomode, so that the output beam has good spatial quality, with a coefficient M² less than 1,5.

5. A pulsed laser according to any one of claims 1 to 4, **characterized in that** the first pump (8) of the power amplifier is an erbium-doped or erbium-ytterbium co-doped optical fiber laser.

6. A pulsed laser according to any one of claims 1 to 5, **characterized in that** the first pump (8) of the power amplifier is directly coupled into the doped core of the power amplifier optical fiber (7).

7. A pulsed laser according to any one of claims 1 to 6, **characterized in that** the first pump (8) of the power amplifier is suitable for pumping the section of power amplifier optical fiber (7) in co-propagation and/or in contra-propagation manner.

8. A pulsed laser according to any one of claims 1 to 7, **characterized in that** the first pump (8) of the power amplifier is suitably for generating at least one second pump wavelength lying in the range 1530 nm to 1565 nm.

9. A pulsed laser according to any one of claims 1 to 8, **characterized in that** the wavelength λ_{P} of the first pump (8) of the power amplifier is adjustable so as to optimize the output pulses spectrally and temporally.

10. A pulsed laser according to any one of claims 1 to 9, **characterized in that** it further includes another pump suitable for optically pumping the section of power amplifier optical fiber (7) at another pump wavelength λ'_{P} lying in the range 970 nm to 990 nm simultaneously with the first pump (8) at the pump wavelength λ_{P} so as to increase the amplification gain in the section of amplifier optical fiber.

11. A laser tool for ophthalmic surgery, the tool comprising an optical fiber pulsed laser according to any one of claims 1 to 10.

12. A laser tool according to claim 11, **characterized in that** the energy of the laser pulses (20) is greater than 100 nJ.

## Patentansprüche

1. Gepulster Laser (10) mit optischer Faser und mit Frequenzdrift zur Verstärkung von Sub-Pikosekunden-Impulsen, deren Dauer weniger als 2 Pikosekunden beträgt, mit guter räumlicher Qualität und hoher Energie, welcher umfasst:
- einen Oszillator (1), der geeignet ist, Lichtimpulse (11) mit einer Dauer im Sub-Pikosekunden-Bereich und mit einer missionswellenlänge, die 1565 bis 1625 nm beträgt, auszusenden;
- einen Faserstrecker (3), der geeignet ist, die Lichtimpulse (11) zeitlich zu strecken;
- eine oder mehrere Vorverstärkungsstufen, die jeweils einen optischen Faserabschnitt (4), der mit Erbium dotiert oder mit Erbium-Ytterbium codotiert ist, und eine pumpe (6), die geeignet ist, den optischen Faserabschnitt (4) zur Vorverstärkung optisch zu pumpen, umfassen;
- mindestens einen Ratenreduzierer (Rate Reducer) (2), der geeignet ist, die Pulsfolgefrequenz der Ausgangs-Laserimpulse (20) so zu reduzieren, dass sie zwischen 10 kHz und 1 MHz liegt;
- Leistungsverstörkungsstufe, die einen optischen Faserabschnitt (7), der mit Erbium dotiert oder mit Erbium-Ytterbium codotiert ist, und eine erste Pumpe (8), die geeignet ist, den optischen Faserabschnitt (7) zur LeistungsVerstärkung optisch zu pumpen, umfasst, wobei die Leistungsverstärkungsstufe geeignet ist, verstärkte Impulse (17) zu erzeugen;
- einen Kompressor (9), der geeignet ist, die verstärkten Lichtimpulse (17) zeitlich wieder zu Ausgangs-Laserimpulsen (20) zu komprimieren;
**dadurch gekennzeichnet, dass**
- die erste Pumpe (8) der optischen Faser (7) zur Leistungsverstärkung geeignet ist, mindestens eine Pumpwellenlänge λp zu erzeugen, die 1530 bis 1565 nm beträgt, so dass Ausgangsimpulse (20) erhalten werden, deren Emissionswellenlänge 1565 bis 1625 nm beträgt und deren Energie 100 nJ bis 100 µJ beträgt.

2. Gepulster Laser (10) nach Anspruch 1, wobei die Länge LF des optischen Faserabschnitts (7) der Leistungsverstärkungsstufe kleiner als etwa zehn Meter ist.

3. Gepulster Laser (10) nach Anspruch 1 oder Anspruch 2, **dadurch gekennzeichnet, dass** der optische Faserabschnitt (7) der Leistungsverstärkungsstufe eine Faser mit großem Kern (Large Mode Area, LMA) ist.

4. Gepulster Laser nach einem der Ansprüche 1 bis 3, **dadurch gekennzeichnet, dass** der Kern der optischen Faser (4) zur Vorverstärkung monomodal ist, so dass das Ausgangsbündel von guter räumlicher Qualität ist, mit einem Koeffizienten M2, der kleiner als 1,5 ist.

5. Gepulster Laser nach einem der Ansprüche 1 bis 4, **dadurch kennzeichnet, dass** die erste Pumpe (8) des Leistungsverstörkers ein Laser mit einer optischen Faser ist, die mit Erbium dotiert oder mit Erbium-Ytterbium codotiert ist.

6. Gepulster Laser nach einem der Ansprüche 1 bis 5, **dadurch gekennzeichnet, dass** die erste Pumpe (8) des Leistungsverstärkers direkt in den dotierten Kern der optischen Faser (7) zur Leistungsverstärkung gekoppelt ist.

7. Gepulster Laser nach einem der Ansprüche 1 bis 6, **dadurch gekennzeichnet, dass** die erste Pumpe (8) des Leistungsverstärkers geeignet ist, den optischen Faserabschnitt (7) des Leistungsverstärkers mit gleicher Ausbreitungsrichtung (Copropagation) und/oder entgegengesetzter Ausbreitungsrichtung (Counterpropagation) zu pumpen.

8. Gepulster Laser nach einem der Ansprüche 1 bis 7, **dadurch gekennzeichnet, dass** die erste Pumpe (8) des Leistungsverstärkers geeignet ist, mindestens eine zweite Pumpwellenlänge zu erzeugen, die 1530 nm bis 1565 nm beträgt.

9. Gepulster Laser nach einem der Ansprüche 1 bis 8, **dadurch gekennzeichnet, dass** die Wellenlänge λp der ersten Pumpe (8) des Leistungsverstärkers so einstellbar ist, dass die Ausgangsimpulse spektral und zeitlich optimiert werden.

10. Gepulster Laser nach einem der Ansprüche 1 bis 9, **dadurch gekennzeichnet, dass** er außerdem eine weitere Pumpe umfasst, die geeignet ist, den optischen Faserabschnitt (7) zur Leistungsverstärkung mit einer anderen Pumwellenlänge λ'p, die 970 bis 990 nm beträgt, gleichzeitig mit der ersten Pumpe (8) mit der Pumpwellenlänge λp optisch zu pumpen, so dass der Verstärkungsfaktor in dem optischen Faserabschnitt zur Verstärkung erhöht wird.

11. Laserwerkzeug für die Augenchirurgie, das einen gepulsten Laser mit optischer Faser nach einem der Ansprüche 1 bis 10 umfasst.

12. Laseriwerkzeug nach Anspruch 11, **dadurch gekennzeichnet, dass** die Energie der Laserimpulse (20) größer als 100 nJ ist.
